# EUROPEAN PATENT SPECIFICATION

(11) **EP 1 207 910 B1**
(45) Date of publication and mention of the grant of the patent: **31.05.2006**
(21) Application number: 00958777.5
(22) Date of filing: 01.09.2000
(51) Int. Cl.: A61K 49/00, A61K 49/04

(54) **Composition comprising diagnostic agents in silica-coated containers**
Zusammensetzung von Diagnostika in Silika-beschichteten Behältnissen
Composition d'agents diagnostiques dans des contenants recouverts de silice

(30) Priority: 03.09.1999 GB 9920758
(43) Date of publication of application: 29.05.2002
(73) Proprietor: GE Healthcare Limited, Little Chalfont Buckinghamshire HP7 9NA (GB)
(72) Inventor: ROWLEY, Neil, J-GE Healthcare Limited, Amersham, Buckinghamshire HP7 9LL (GB); CANNING, Lewis Reuben, GE Healthcare Limited, Amersham, Buckinghamshire HP7 9LL (GB)
(74) Representative: Canning, Lewis R.
(86) International application number: PCT/GB2000/003373
(87) International publication number: WO 2001/017567

(56) References cited:
- WO-A-99/08941
- WO-A1-96/26163
- DE-A1- 19 801 861
- US-A- 5 565 248
- US-A- 5 612 103

## Description

### Summary of the Invention

The present invention relates to improved containers for diagnostic agents, which are metal complex contrast agents for MRI or X-ray imaging, or hyperpolarised materials, where the container has an internal coating of silica (ie. silicon dioxide or SiO₂). The silica coating is preferably deposited by a plasma chemical vapour deposition (PCVD) process.

### Field of the Invention

The present invention relates to diagnostic agents, especially for *in vivo* use, in a coated container, where the container has an internal coating of silica coating on the surface(s) which are in contact with the diagnostic agent.

### Background to the Invention

US 4385086 (1983) discloses that a variety of materials (eg. soda glass, ceramics and metals) can be coated with highly oxidised silicon to prevent the leaching of metal ions from the material.

FR 2697014 A1 (1994) discloses the silica coating of the bottles, flasks, ampoules etc. for use with food or liquid pharmaceutical products to reduce leaching of metals into the liquid contents of the container.

DE 29609958 U1 discloses that glass containers having an internal coating of SiO₂ prepared by PCVD are useful for the storage of pharmaceutical or diagnostic solutions.

JP 11-99192A discloses that silica-coated vials (prepared by a chemical coating and pyrolysis method), are useful to prevent adsorption of radiopharmaceutical products such as ²⁰¹Tl solution to the surface of the glass. The silica coating of these vials is manufactured by the method described in JP 2815595 B which involves treating the glass surface with a silyl tetraisocyanate vapour in a carrier gas, followed by heating at high temperatures. JP 2815595 B also discloses that such a silica coating is useful to prevent leaching of impurities such as alkali from the glass into medical products.

US 5612103 discloses the use of coatings of deuterated polymers to inhibit the depolarisation of hyperpolarised gases within a container. WO 99/08941 discloses the use of glass vessels coated with a sol-gel, preferably an aluminosilicate glass, for the same purpose. WO 99/17304 discloses the use of a container made of a special glass having a low iron content for the same purpose.

### Summary of the Invention

The present invention relates to silica-coated containers in combination with the following categories of diagnostic agents:
(i) lyophilised kits or liquid or solution formulations for the preparation of MRI contrast agents which comprise metal complexes of paramagnetic metal ions;
(ii) lyophilised kits or liquid or solution formulations of X-ray contrast agents which comprise metal complexes of radiopaque metal ions;
(iii) hyperpolarised gases such as ¹²⁹Xe or ³He or other hyperpolarised materials.

### Detailed Description of the Invention

The present invention relates to a composition comprising a diagnostic agent in a container which has a silica coating on the inner surface. Suitable such containers are commercially available, e.g. a silica-coated vial called Silicoat is available from Fuji Glass KK, and a silica-coated vial called Type I Plus is available from Schott Glas. The Type I Plus vial is prepared by a plasma chemical vapour deposition (PCVD) process.

Other containers can be coated with silica using known methods, where the silicon-containing layer is deposited from either gas phase or liquid phase contact with the container surface(s), with optional pyrolysis and/or oxidation to convert the deposited silicon-containing layer to SiO₂. Such methods are known in the art. Using either approach, irregularly-shaped containers can be coated. Examples of gas phase deposition are PCVD, and the process of JP 2815595 B which uses silyl tetraisocyanate vapour in carrier gas. The latter process delivers the silicon-containing layer in a single step, with pyrolysis of silyl tetraisocyanate required to give the final product, i.e. the coated vial. Depending on the efficiency of the heat transfer, the coating layer may not be pure SiO₂ but perhaps contain carbon or nitrogen. The silica-coated vial prepared by PCVD has advantages over the disclosures of JP 2815595 B and JP 11-99192A, because the SiO₂ layer prepared by PCVD is in fact developed by multiple exposure to the vapour phase silicon reagent. The result is a much more uniform layer of high purity SiO₂, which is mechanically sound and resistant to abrasion etc. Hence PCVD is a preferred process for use in containers of the present invention.

The term "diagnostic agent" as used herein means either a hyperpolarised material or a non-radioactive metal complex which is an MRI contrast agent or an X-ray contrast agent. The "hyperpolarised material" can be a hyperpolarised gas, such as ¹²⁹Xe or ³He, or a ¹³C- or ¹⁵N-enriched labelled molecule or a ¹⁹F- or ³¹P- containing molecule where the MRI active nucleus of interest has been hyperpolarised by a polarisation transfer process. The silica coatings of the present invention are believed to be especially useful for hyperpolarised ¹²⁹Xe compositions, since xenon has low solubility in silica. The diagnostic agent may be used for *in vivo* and/or *in vitro* diagnosis. The metal complex contrast agents of the present invention are preferably for *in vivo* use.

The term "metal complex" as used herein means a coordination complex of a metal (M) with an organic ligand (L). This is to be contrasted with an uncomplexed or free metal ion e.g. the monovalent thallium cation T1⁺ The term 'organic ligand' as used herein means a carbon-containing compound which comprises at least one heteroatom suitable for coordination to a metal, such as N, O, S, P or Se, or combinations thereof. Examples of organic ligands are amines, hydrazines, ethers such as crown ethers, thiols or thioethers, oximes, phosphines, amides, pyridines or other heterocyclic molecules such as quinolines, aminocarboxylate ligands such as DTPA, DOTA or HEDTA. The metal donor atoms can be arranged together to form chelating agents or polydentate ligands such as diaminedioximes, hydroxyquinolines, diaminedithiols, diamidedithiols; or macrocyclic ligands such as DOTA, and many more combinations as is well known in the art. MRI contrast agents are typically metal complexes, where the metal is a paramagnetic metal ion such as gadolinium (III) of aminocarboxylate ligands such as DTPA or DOTA. X-ray contrast agents can also be metal complexes, where the metal is radiopaque, such as bismuth or tungsten.

When the diagnostic agent is or comprises a metal complex, if leached metal ions (M') such as aluminium or sodium, are leached from glass into the product, these leached metal ions may adversely affect the product in a manner which goes beyond the simple presence of M' as an impurity:

(i) MLₙ + M' → M'L_{q} + M ligand exchange or transmetallation

(ii) L + M' → M'L_{q} complexation

where:
M is the metal of the desired metal complex product,
L is the organic ligand;
M' is the leached metal ion;
MLₙ is the metal complex product, which may comprise 2 or more different organic ligands L;
n is number of ligands (L) attached to M and is an integer of value 1 to 8;
M'L_{q} is the metal complex impurity;
q is number of ligands (L) attached to M' and is an integer of value 1 to 8.

Process (i) can occur when the leached metal or metals (M') have greater affinity for one or more of the organic ligands (L) than the metal (M) of the contrast agent product. In addition to, or instead of equation (i), complexation (ii) may also occur. This leads to the presence of undesirable M'Lq impurities in the product MLₙ.

When L is a multidentate ligand, such as a chelating agent the number of metal donor sites (A) per ligand (L) may be 2, 3, 4, 5, 6 or 8 typically. In that case, a process which is a special case of equation (i) above could occur as follows: 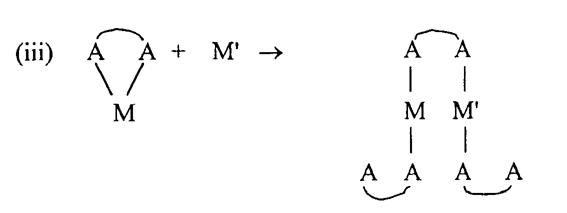 where: the free A donors can complex to further M/ M' atoms etc.
note: the curved lines represent the chain of atoms linking the A groups.

leading to dimeric or oligomeric binuclear or polynuclear metal complexes involving both M and M'. The leached metal (M') may be less amenable to chelation by polydentate ligand (L), and hence favour such polynuclear species, even when M does not. This could result when the energetics are less favourable, e.g. M' is too small for two A groups to coordinate without undue steric interactions. In this way, a single M' atom could potentially generate a polynuclear or oligomeric species which comprises several M atoms. Clearly, the greater the denticity of the ligand L (i.e. the greater the number of A metal donor sites), the greater the potential complexity of the product.

The presence of such species may present impurity or manufacturing or irreproducibility problems due to vial-to-vial variations, or toxicity problems due to the impurity species or particulate problems when insoluble materials result, eg. when M'Lq is highly insoluble. Such insoluble impurities may also potentially serve to promote coprecipitation of the desired product MLₙ. Particulate problems would be a serious safety problem for products intended for human injection. Impurity species may also adversely affect product imaging performance by e.g. localising in undesirable background areas *in vivo* which adversely impact the image to be made.

In the light of the above, it can be seen that the influence of leachable metal ions (M'), can have effects which go far beyond simply the presence of metal ion impurities alone in solution. This is important for metal complex contrast agent products, and is not recognised by JP 11-99192A which makes no specific reference to metal complexes. The ²⁰¹T1 radioisotope taught by JP 11-99192 A is an uncomplexed radiometal in the chemical form of the T1(I) cation Tl⁺. The teaching of JP 11-99192 A relates only to adsorption effects *via* an ion exchange mechanism for the ²⁰¹T1 cation T1⁺ with the non-radioactive Na⁺ and K⁺ ions of the glass container walls. The main thrust of JP 11-99192 A is to a radiopharmaceutical vial having reversed text characters on the surface of the container.

For uncoated glass containers, the leaching of metal ions from the glass can potentially be overcome by washing with dilute aqueous acid solutions (to remove relatively labile leachable metal ions), following by rinsing and (optionally) drying steps, before the container is loaded with product. The layer of SiO₂ suppresses any such leaching of metal ions (M'), and hence obviates the need for any such washing steps. This is particularly important for diagnostic products intended for human use, especially for human injection, since these washing steps must be done in a sterile manner, hence although such steps may be straightforward, their removal represents a significant improvement.

Hyperpolarised materials are non-radioactive materials which have a finite lifetime due to the decay process from the hyperpolarised state to the ground state, i.e. depolarisation. The rate of depolarisation is believed to be increased in the presence of paramagnetic species, especially molecular oxygen, or paramagnetic metal ions e.g. Fe³⁺. The composition of the present invention where the diagnostic agent is a hyperpolarised material, therefore provides improved containers where an inert layer of essentially pure SiO₂ is interposed between the hyperpolarised material and the normal walls of the container.

The SiO₂ layer is free from any metallic ions or paramagnetic species and is of high chemical purity, especially when it is deposited by CPVD. It is hence expected to represent an improvement over prior art approaches to increasing the lifetime of hyperpolarised species.

Silica coating techniques based on gas phase deposition, eg. PCVD, can be adapted to coat the inner surfaces of containers or vessels of almost any shape or size. Hence it is anticipated that the container compositions of the present invention can be applied to production apparatus as well as storage and transport vessels. The silica coating can also readily be applied to non-rigid, flexible materials such as thin plastics. Such containers could be used e.g. for containing doses of hyperpolarised gases such as ¹²⁹Xe or ³He in plastic bags with an inner silica coating, as unit doses for human administration by inhalation.

Example 1 shows that a silica-coated vial (the Type 1 Plus vial) does prevent the leaching of silicon, sodium, aluminium and boron ions from the glass, even under stress conditions.

### Experimental

### Example 1

Groups of 10 Type I Plus vials (Schott Glas) were subjected to a series of stress tests to demonstrate the robustness of the silica coating with respect to leachable ions. The basic test was the resistance of the coating to the leaching of cations when autoclaved with 0.04M aqueous HCl. This test was performed after vials were exposed to the following stress conditions:
1. vials were washed, and then pyrogen baked. 2ml of 0.04M HCl was added and the vials sealed. Test vials were autoclaved, then stored upright at 40°C before testing for leachable cations.
2. vials were stored for 6 weeks at -196°C, then washed and pyrogen baked. 2ml of 0.04M HCl was added to each vial, and the vials were then sealed, autoclaved and tested for leachable cations.
3. as for test 2, except that the vials were stored at -70°C, -20°C, +20°C and +40°C/75% relative humidity.
4. further tests included: vials pyrogen baked 3 times; vials containing 0.04M HCl autoclaved three times; vials gamma irradiated (35.4 - 36.2 kGy dose).

All test solutions were measured by ICP for silicon, sodium, aluminium and boron, those cations considered to be most leachable from the vial surface. The results are given in Table 1.

**Table 1**

| | | | | |
|---|---|---|---|---|
| **Test Number** | **Si** | **Na** | **Al** | **B** |
| 1 | 0.149 | Nd | 0.006 | Nd |
| 2 | 0.163 | Nd | Nd | Nd |
| 3 -70°C | 0.167 | Nd | Nd | Nd |
| -20°C | 0.193 | 0.005 | 0.002 | 0.002 |
| +20°C | 0.193 | 0.009 | 0.005 | 0.003 |
| +40°C | 0.236 | 0.006 | 0.002 | 0.002 |
| 4 bake | 0.110 | Nd | 0.010 | Nd |
| X3 | 0.378 | 0.012 | Nd | 0.006 |
| Gamma | 0.102 | 0.003 | Nd | Nd |

| | | | | |
|---|---|---|---|---|
| Note: each table entry is the mean of 12 batch runs, each batch of 10 vials (i.e. 120 vials tested), expressed in µg/cm³ of test solution. | | | | |
| Nd = not detected. Detection limits (in µg/cm³): B - 0.004 Si - 0.003 Na - 0.004 Al- 0.004 | | | | |

## Claims

1. A composition which comprises a diagnostic agent in a container which has a silica coating on the inner surface, **characterised in that** the diagnostic agent comprises a non-radioactive metal complex or a hyperpolarised material;
wherein the hyperpolarised material comprise an MRI active nucleus which has been hyperpolarised by a polarisation transfer process.

2. The composition of claim 1 where the diagnostic agent comprises a non-radioactive metal complex.

3. The composition of claim 2 where the metal complex is an MRI contrast agent.

4. The composition of claim 2 where the metal complex is an X-ray contrast agent

5. The composition of claim 1 where the diagnostic agent comprises a hyperpolarised material.

6. The composition of claim 5 where the hyperpolarised material comprises hyperpolarised ¹²⁹Xe or ³He gas.

7. The composition of claim 5 where the hyperpolarised material comprises one or more hyperpolarised ¹³C atoms.

8. The composition of claims 1 to 7 where the silica coating is deposited by plasma chemical vapour deposition (PCVD).

9. The composition of claims 1 to 8 where the silica coating comprises pure SiO₂.

## Patentansprüche

1. Zusammensetzung, umfassend ein Diagnosemittel in einem Behälter, der eine Silica-Beschichtung auf der inneren Oberfläche aufweist, **dadurch gekennzeichnet, dass** das Diagnosemittel einen nicht-radioaktiven Metallkomplex oder ein hyperpolarisiertes Material umfasst;
wobei das hyperpolarisierte Material einen MRI-aktiven Nukleus umfasst, der durch einen Polarisationstransferprozess hyperpolarisiert worden ist.

2. Zusammensetzung nach Anspruch 1, bei der das Diagnosemittel einen nicht-radioaktiven Metallkomplex umfasst.

3. Zusammensetzung nach Anspruch 2, bei der der Metallkomplex ein MRI-Kontrastmittel ist.

4. Zusammensetzung nach Anspruch 2, bei der der Metallkomplex ein Röntgenstrahlen-Kontrastmittel ist.

5. Zusammensetzung nach Anspruch 1, bei der das Diagnosemittel ein hyperpolarisiertes Material umfasst.

6. Zusammensetzung nach Anspruch 5, bei der das hyperpolarisierte Material hyperpolarisiertes ¹²⁹Xe- oder ³He-Gas umfasst.

7. Zusammensetzung nach Anspruch 5, bei der das hyperpolarisierte Material einen oder mehrere hyperpolarisierte ¹³C-Atome umfasst.

8. Zusammensetzung nach den Ansprüchen 1 bis 7, bei der die Silica-Beschichtung durch chemische Abscheidung aus der Plasma-Gasphase (PCVD) abgeschieden wird.

9. Zusammensetzung nach den Ansprüchen 1 bis 8, bei der die Silica-Beschichtung reines SiO₂ umfasst.

## Revendications

1. Composition qui comprend un agent de diagnostic dans un conteneur qui présente un revêtement de silice sur la surface interne, **caractérisée en ce que** l'agent de diagnostic comprend un complexe de métal non radioactif ou un matériau hyperpolarisé ;
dans laquelle le matériau hyperpolarisé comprend un noyau actif d'imagerie IRM qui a été hyperpolarisé par un processus de transfert de polarisation.

2. Composition selon la revendication 1, dans laquelle l'agent de diagnostic comprend un complexe de métal non radioactif.

3. Composition selon la revendication 2, dans laquelle le complexe de métal est un agent de contraste d'imagerie IRM.

4. Composition selon la revendication 2, dans laquelle le complexe de métal est un agent de contraste de radiographie.

5. Composition selon la revendication 1, dans laquelle l'agent de diagnostic comprend un matériau hyperpolarisé.

6. Composition selon la revendication 5, dans laquelle le matériau hyperpolarisé comprend du gaz ¹²⁹Xe ou ³He hyperpolarisé.

7. Composition selon la revendication 5, dans laquelle le matériau hyperpolarisé comprend un ou plusieurs atomes de ¹³C hyperpolarisé.

8. Composition selon les revendications 1 à 7, dans laquelle le revêtement de silice est déposé par dépôt chimique en phase vapeur sous plasma (PCVD).

9. Composition selon les revendications 1 à 8, dans laquelle le revêtement de silice comprend de la silice pure SiO₂.
